# EUROPEAN PATENT SPECIFICATION

(11) **EP 4 258 994 B1**
(45) Date of publication and mention of the grant of the patent: **25.11.2026**
(21) Application number: 21904479.9
(22) Date of filing: 10.12.2021
(51) Int. Cl.: A61B 6/00, A61B 6/03, G06T 11/00

(54) **SYSTEMS FOR LASER-INDUCED CALCIUM FRACTURES**
SYSTEME FÜR LASERINDUZIERTE CALCIUMFRAKTUREN
SYSTÈMES DE FRACTURES DE CALCIUM INDUITES PAR LASER

(30) Priority: 11.12.2020 US 202063124357 P
(43) Date of publication of application: 18.10.2023
(73) Proprietor: Research Development Foundation, Carson City, NV 89703 (US)
(72) Inventor: MILNER, Thomas, E., Irvine, CA 92617 (US); FELDMAN, Marc, D., San Antonio, TX 78248 (US); KATTA, Nitesh, Irvine, CA 92612 (US); JENNEY, Scott, Austin, TX 78712 (US); CABE, Andrew, San Antonio, TX 78229 (US); GRUSLOVA, Aleksandra, Borisovna, San Antonio, TX 78240 (US)
(74) Representative: Vossius & Partner Patentanwälte Rechtsanwälte mbB
(86) International application number: PCT/US2021/062838
(87) International publication number: WO 2022/125919

(56) References cited:
- EP-A2- 0 182 689
- WO-A1-2020/089876
- WO-A1-2023/076241
- US-A- 5 944 687
- US-A1- 2006 190 022
- US-A1- 2011 301 418
- US-A1- 2014 031 800
- US-A1- 2016 184 022
- US-A1- 2017 265 942
- US-A1- 2018 098 779
- US-A1- 2020 129 195

## Description

### BACKGROUND INFORMATION

Coronary artery atherosclerosis is the most common type of cardiovascular disease and results in the death of hundreds of thousands of people in the United States each year. Calcium in atherosclerosis is common in coronary artery disease (CAD), and is problematic during coronary intervention. Calcium reduces arterial compliance and can compromise cardiac output and complicate cardiovascular interventions. For example, calcium increases the complexities of treatment because it prevents full stent expansion which can lead to stent thrombosis (heart attacks) with a high death rate.

Solutions which are currently used clinically to increase vessel compliance and deal with excessive calcium include high pressure balloon inflation, and calcium scoring with cutting balloons. However, these approaches are often unsuccessful for a variety of reasons. Coronary atherectomy with both rotational atherectomy systems (*e.g.* Rotablator^{™}) and orbital atherectomy are suited for removing luminal superficial calcium. However, these approaches do not address deeper calcium and therefore do not always increase vessel compliance sufficiently to assure full stent expansion. These techniques are also technically complex, time consuming and can introduce increased risk since they send cut debris into the microcirculation which can result in myocardial infarction during the procedure. Thus, dealing with the calcium burden of atherosclerosis presents is a safe and efficacious manner is a major clinical challenge for cardiovascular health and therapy including placement of fully expanded stents. US2014/0031800 Al relates to an intravascular catheter for treating calcified plaque in blood vessels and discloses a catheter having optical fibers, a laser source, and, in some embodiments, a balloon arrangement.

Intravascular lithotripsy techniques based on kidney stone treatment have been developed using electrodes inside a balloon catheter. The electrodes vaporize the fluid within the balloon generating sonic pressure waves that travel through soft vascular tissue and selectively fracture calcium in the vessel wall. The high difference in density and mechanical properties between calcium and soft tissue allows the sonic pressure to fracture calcium while leaving soft tissue undamaged. However, the use of electrodes limits the amount of energy available and the ability to control spatially and temporally the delivery of energy to vaporize the fluid and induce calcium fracture. The electrical approaches also result in large voltage spikes requiring pacing the heart with each delivered electric pulse, which is not ideal.

Accordingly, systems and methods are desired that overcome these and other limitations associated with existing systems and methods.

### SUMMARY

An urgent need is recognized for the ability to effectively fracture intravascular calcium for treatment of patient conditions, including atherosclerosis and other coronary diseases. Similarly, a need to decalcify heart valves, and the aorta is also recognized.

The invention is defined in the appended set of claims. Methods mentioned hereinafter do not form part of the present invention.

Exemplary embodiments of the present disclosure provide unique advantages over existing systems and methods. For example, it is believed that more effective treatment can be provided by utilizing electromagnetic energy (including for example, laser energy) to generate sonic pressure within an expandable member such as a balloon. Laser generated pressure amplitudes are an order of magnitude greater than electrode generated pressure. In addition, laser radiation allows flexible temporal and spatial control over shock-wave generation. Advantages of pressure amplitude, temporal and spatial control may be utilized to provide greater and more efficient calcium fracturing.

In addition, laser generation of a shock wave also has the advantage of finer spatial and temporal control of the cavitation or bubble creation in the liquid contained within the balloon that generates the pressure. Specific bubble shapes with preset arrival times may also be created with varying duration of the laser pulse that can allow for more predictable and improved calcium fracture. In addition, laser approaches allow time generation of secondary pulses that can provide therapeutic benefits. While existing techniques may use optical imaging to verify efficacy of calcium fracture after therapy, exemplary embodiments of the present disclosure can provide imaging during calcium fracture to monitor efficacy of calcium fracture in real-time. Specific embodiments of the present disclosure may be used for treatment of calcified aortic stenosis to decalcify valve leaflets and delay need for aortic valve replacement (AVR) or transcatheter aortic valve replacement (TAVR). Approaches described herein can also be used to create calcium fracture in the aorta, improving elastic recoil and thus improving blood supply during diastole to the microcirculation in varying disease conditions.

Exemplary embodiments include an apparatus configured to fracture coronary calcium, where the apparatus comprises: an expandable member; a laser light source; and an optical fiber coupled to the laser light source, where: the optical fiber comprises one or more emission regions configured to emit electromagnetic energy from the laser light source from the optical fiber; and emission of electromagnetic energy from the one or more emission regions is configured to create fractures in the coronary calcium. In certain embodiments, the expandable member comprises a fluid, and the emission of electromagnetic energy from the emission regions is configured to create fractures in the coronary calcium by generating ultrasonic waves in the fluid. In particular embodiments, the one or more emission regions are configured as conical reliefs in the optical fiber. In some embodiments, the optical fiber is a first optical fiber; the apparatus further comprises a plurality of optical fibers; each optical fiber of the plurality of optical fibers comprises one or more emission regions configured to emit electromagnetic energy in a radial pattern from each optical fiber. In specific embodiments, the expandable member is a balloon.

In certain embodiments, the expandable member is configured to be expanded via a fluid contained within the expandable member. Particular embodiments further comprise a first port configured to deliver the fluid to the expandable member. Some embodiments further comprise a second port configured to drain the fluid from the expandable member. In specific embodiments, the fluid is configured to absorb electromagnetic energy from the optical fiber, generate an acoustic wave and propagate to the calcium. In certain embodiments, the fluid is a saline fluid. In particular embodiments, the optical fiber is configured to emit the electromagnetic energy in a radial pattern. In some embodiments, the electromagnetic energy is emitted at a wavelength of approximately 2 µm. In specific embodiments, the electromagnetic energy is emitted at a wavelength between 1.5 µm and 2.5 µm. Certain embodiments further comprise an intravascular imaging device. In particular embodiments, the intravascular imaging device is an intravascular ultrasound (IVUS) device. In some embodiments, the intravascular imaging device is an optical coherence tomography imaging (OCT) device.

Exemplary embodiments include a method of fracturing calcium in an artery, where the method comprises: inserting a catheter into an artery; and emitting electromagnetic energy from the catheter, where: calcium is located within the artery, the catheter comprises a laser light source and an optical fiber; fluid surrounds the optical fiber; and the electromagnetic energy is generated by the laser light source; and absorbed electromagnetic energy in the fluid surrounding the optical fiber creates an acoustic wave that enters the arterial wall and fractures the calcium.

In certain embodiments, the catheter comprises an expandable member, and the method further comprises expanding the expandable member. In particular embodiments, the expandable member is expanded after the catheter is inserted into the artery and prior to emitting electromagnetic energy from the catheter. In some embodiments, the expandable member is expanded to conform to the surface of the calcium located within the artery. In specific embodiments, the expandable member is expanded via a fluid contained within the expandable member. In certain embodiments, the electromagnetic energy emitted from the catheter is absorbed by fluid surrounding the optical fiber and propagates into the calcium. In particular embodiments, the electromagnetic energy emitted from the catheter causes cavitation in the fluid contained within the expandable member. In some embodiments, the cavitation creates ultrasonic waves in the fluid contained within the expandable member. In specific embodiments, the ultrasonic waves create fractures in the calcium located within the artery. In certain embodiments, the calcium comprises inhomogeneities, and the fractures are formed along the inhomogeneities in the calcium. In particular embodiments, fracturing the calcium increases the compliance of the artery. In some embodiments, the electromagnetic energy is emitted at a wavelength of approximately 2 µm. In specific embodiments, the electromagnetic energy is emitted at a wavelength between 1.5 µm and 2.5 µm. Certain embodiments further comprise imaging the artery while fracturing the calcium, and particular embodiments further comprise imaging the artery prior to fracturing the calcium.

Certain embodiments include an apparatus configured to fracture coronary calcium, where the apparatus comprises: an intravascular imaging device; an expandable member; a laser light source configured to emit electromagnetic energy; and an optical fiber coupled to the laser light source, and where: the optical fiber comprises a proximal end and a distal end; and the optical fiber is configured to emit electromagnetic energy from the laser light source from the distal end of the optical fiber. In particular embodiments, the expandable member comprises a fluid; and the electromagnetic energy from the distal end of the fiber is configured to create fractures in the coronary calcium by generating ultrasonic waves in the fluid. In some embodiments, the expandable member is a balloon. In specific embodiments, the expandable member is configured to be expanded via a fluid contained within the expandable member. Certain embodiments further comprise a first port configured to deliver the fluid to the expandable member. Particular embodiments further comprise a second port configured to drain the fluid from the expandable member. In some embodiments, the second port is further configured to evacuate vapor bubbles from the expandable member. In specific embodiments, the fluid is configured to absorb electromagnetic energy from the optical fiber, generate an acoustic wave and propagate to the calcium. In certain embodiments, the fluid is indocyanine green (ICG). In particular embodiments, the electromagnetic energy is emitted at a wavelength between 790-810 nanometers (nm). In specific embodiments, the electromagnetic energy is emitted at a wavelength of approximately 793 nm.

In certain embodiments, the electromagnetic energy emitted from the optical fiber is less than 1.0 kilowatt (kW). In particular embodiments, the electromagnetic energy emitted from the optical fiber at approximately 0.6 kW. The laser light source is a diode laser. In specific embodiments, the intravascular imaging device is an intravascular ultrasound (IVUS) device. In certain embodiments, the intravascular imaging device is an optical coherence tomography imaging (OCT) device. In particular embodiments, the intravascular imaging device has an outer diameter of less than 2.0 millimeters (mm). In some embodiments, the intravascular imaging device has an outer diameter of approximately 1.2 millimeters mm.

In the following disclosure, the term "coupled" is defined as connected, although not necessarily directly, and not necessarily mechanically.

The use of the word "a" or "an" when used in conjunction with the term "comprising" in the claims and/or the specification may mean "one," but it is also consistent with the meaning of "one or more" or "at least one." The terms "about" and "approximately" mean, in general, the stated value plus or minus 5%. The use of the term "or" in the claims is used to mean "and/or" unless explicitly indicated to refer to alternatives only or the alternative are mutually exclusive, although the disclosure supports a definition that refers to only alternatives and "and/or."

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a method or device that "comprises," "has," "includes" or "contains" one or more steps or elements, possesses those one or more steps or elements, but is not limited to possessing only those one or more elements. Likewise, a step of a method or an element of a device that "comprises," "has," "includes" or "contains" one or more features, possesses those one or more features, but is not limited to possessing only those one or more features. Furthermore, a device or structure that is configured in a certain way is configured in at least that way, but may also be configured in ways that are not listed.

Other objects, features and advantages of the present invention will become apparent from the following detailed description. It should be understood, however, that the detailed description and the specific examples, while indicating specific embodiments of the invention, are given by way of illustration only, since various changes and modifications within then scope of the invention will be apparent to those skilled in the art from this detailed description.

### BRIEF DESCRIPTION OF THE DRAWINGS

The following drawings form part of the present specification and are included to further demonstrate certain aspects of the present disclosure. The invention may be better understood by reference to one of these drawings in combination with the detailed description of specific embodiments presented herein.
**FIG. 1** shows a schematic view of an artery with a guidewire for use with an apparatus according to an exemplary embodiment.
**FIG. 2** shows a schematic view of an exemplary embodiment according to the present disclosure during an initial stage of use.
**FIG. 3****.** shows a schematic view of a portion of the embodiment of FIG. 1 during use.
**FIG. 4** shows a schematic view of a portion of the embodiment of FIG. 1 during use.
**FIG. 5** shows schematic end view of an exemplary embodiment according to the present disclosure.
**FIG. 6** shows a schematic view of a portion of the embodiment of FIG. 5 during use.
**FIG. 7** shows a schematic view of a portion of the embodiment of FIG. 5 during use.
**FIG. 8** shows a schematic view of a portion of the embodiment of FIG. 5 during use.
**FIG. 9** shows a schematic view of a portion of the embodiment of FIG. 5 during use.
**FIG. 10** shows an end view of an exemplary embodiment according to the present disclosure.
**FIG. 11** shows an exemplary dimensional drawing of the embodiment of FIG. 10.
**FIG. 12** shows a graph of peak amplitude of the pressure as a function of fluence rate for various techniques.
**FIG. 13** shows a graph of pressure versus volume compliance curves measured during testing of exemplary embodiments of the present disclosure.
**FIG. 14** shows a graph of pressure versus volume compliance curves measured during testing of exemplary embodiments of the present disclosure.
**FIG. 15** shows an optical coherence tomography (OCT) image of an artery before treatment according to exemplary embodiments of the present disclosure.
**FIG. 16** shows an optical coherence tomography (OCT) image of an artery after treatment according to exemplary embodiments of the present disclosure.
**FIG. 17** shows a graph indicating molar extinction coefficient versus wavelength according to an exemplary embodiment of the present disclosure.
**FIG. 18** shows a graph indicating pressure versus Joules per pulse according to an exemplary embodiment of the present disclosure.
**FIG. 19** shows a graph indicating molar extinction coefficient versus wavelength according to an exemplary embodiment of the present disclosure.
**FIG. 20** shows a graph indicating absorbance versus wavelength according to an exemplary embodiment of the present disclosure.
**FIG. 21** shows a graph indicating nanorod optical density versus wavelength according to an exemplary embodiment of the present disclosure.
**FIG. 22** shows a schematic view of an exemplary embodiment according to the present disclosure during use.
**FIG. 23** shows a section view of the embodiment of FIG. 22.
**FIG. 24** shows a schematic view of an embodiment of an optical fiber of the embodiment of FIG. 22.
**FIG. 25** shows OCT images of an ex vivo human artery before and after undergoing laser induced lithotripsy procedures.
**FIG. 26** shows images of different subjects before and after undergoing laser induced lithotripsy procedures.
**FIG. 27** shows before and after micro-CT images of a human ex vivo artery demonstrating laser induced fracture.
**FIG. 28** shows a graph of pressure versus energy for different pulse durations in different fluids for laser induced lithotripsy procedures.
**FIG. 29** shows stenosis in a rabbit model and laser induced shockwave fractures in ex vivo human arteries.
**FIG. 30** shows an embodiment of laser light source comprising a plurality of diode lasers.
**FIG. 31** shows a graph of absorption coefficient versus wavelength for different concentrations of indocyanine green (ICG) in a saline water solution.
**FIG. 32** shows a graph of absorption coefficient versus wavelength for the same concentration of ICG in different solutions.

### DETAILED DESCRIPTION OF ILLUSTRATIVE EMBODIMENTS

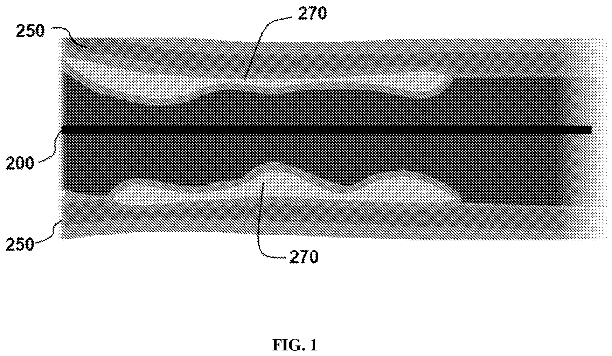

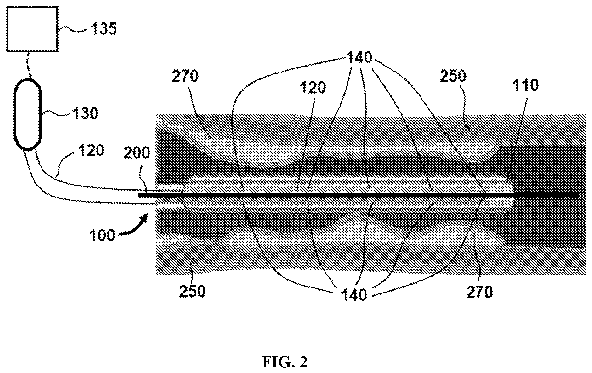

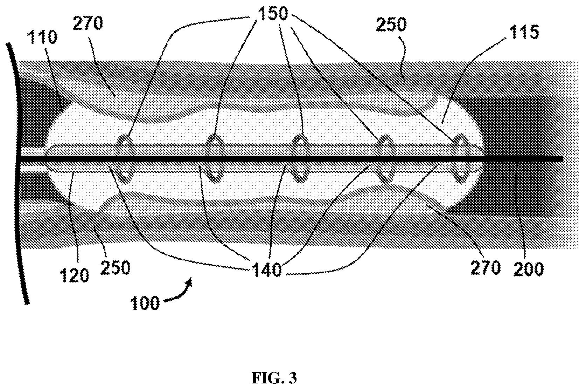

Exemplary embodiments of the present disclosure include apparatus and methods for fracturing arterial calcium, including for example calcium in a coronary artery. Referring initially to FIGS. 1-4 an overview of an exemplary apparatus and method of use are demonstrated. For purposes of clarity, not all features shown in each figure are labeled with reference numbers in every figure. In FIG. 1, a guide wire 200 has been inserted into a coronary artery 250 with calcium 270 located within artery 250. In FIG. 2 a catheter apparatus 100 has been inserted over guidewire 200 into artery 250. In the embodiment shown, apparatus 100 comprises an expandable member 110 *(e.g.* a balloon) and an optical fiber 120 coupled to a laser light source 130. In the illustrated embodiment, optical fiber 120 comprises one or more emission points 140 configured to emit electromagnetic energy 150 (shown in FIG. 3) from laser light source 130 in a radial pattern from optical fiber 120. In certain embodiments emission points 140 may be configured as conical reliefs or ends of optical fiber 120. In other embodiments, emission points 140 may be configured as beveled, angled or flat reliefs or ends of optical fiber 120. In the embodiment shown, apparatus 100 comprises a control system 135 configured to control operational parameters of apparatus 100, including for example, the operation of laser light source 130 (*e.g.* laser pulse duration, frequency, amplitude *et al.*)*.*

In the embodiment shown in FIG. 3, expandable member 110 has been expanded within artery 250 via a fluid 115 (*e.g.* a saline fluid) that is pressurized within expandable member 110. In the embodiment shown, expandable member 110 has been expanded after apparatus 100 has been inserted into artery 250 and prior to emitting electromagnetic energy 150 from apparatus 100. Electromagnetic energy 150 creates cavitation 155 (*e.g.* bubbles) in fluid 115 which generates ultrasonic waves 125 from the formation and collapse of the bubbles 155 in fluid 115. In certain embodiments, expandable member 110 can be configured as a large balloon configured for treatment of the distal aorta in order to increase compliance of the aorta in elderly patients with resistant systolic hypertension, and to increase elastic recoil during diastole to improve blood flow to the microcirculation.

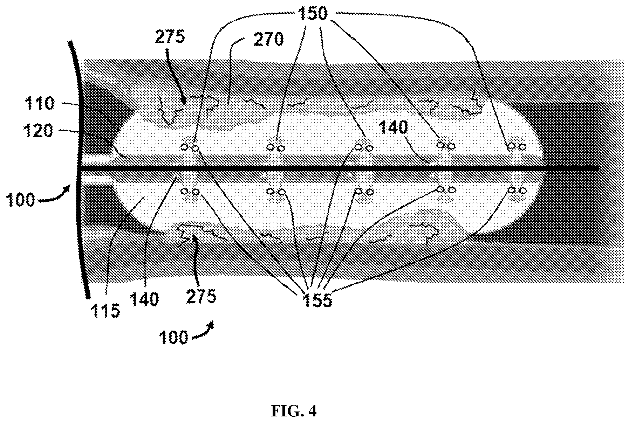

As shown in FIG. 4, ultrasonic waves 125 propagate through fluid 115 and create fractures 275 only in calcium 270 without damaging the vessel walls, since the vessel walls are more elastic than the calcium plaque. In exemplary embodiments, fractures 275 are created along inhomogeneities in calcium 270 and/or in calcium-hard-soft tissue interfaces. Fracturing of calcium 270 increases compliance of artery 250, allowing artery 250 to more easily expand and contract with changes in pressure.

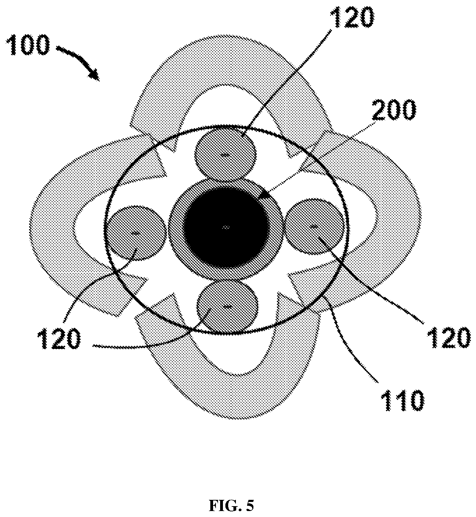

Referring now to FIGS. 5-11, another embodiment of the present disclosure is shown during use. This embodiment is similar to the previously-described embodiment, but includes multiple optical fibers. Although not shown in FIGS. 5-11, it is understood that this embodiment includes components shown in FIGS. 1-4, including for example, laser light source 130 and control system 135.

Referring initially to FIG. 5, an end view of apparatus 100 is shown with four optical fibers 120. While four optical fibers 120 are shown in the illustrated embodiment, it is understood that other embodiments may comprise more or fewer optical fibers than the four shown in this embodiment.

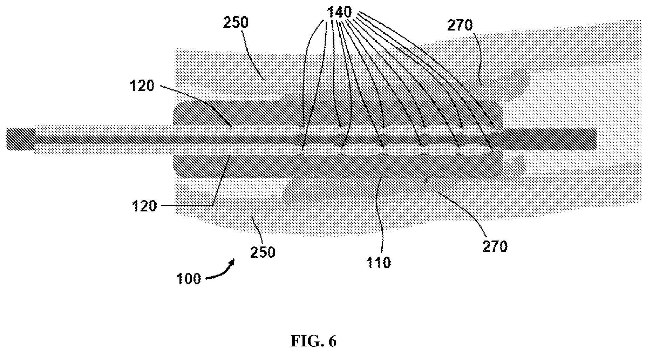

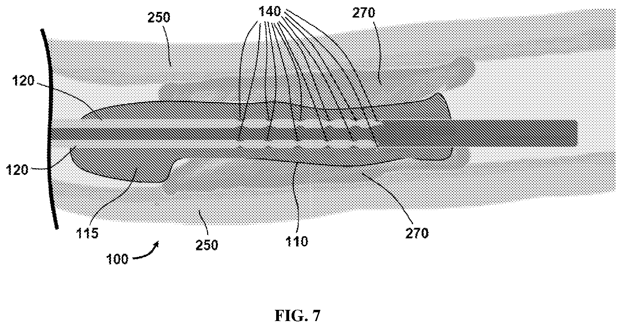

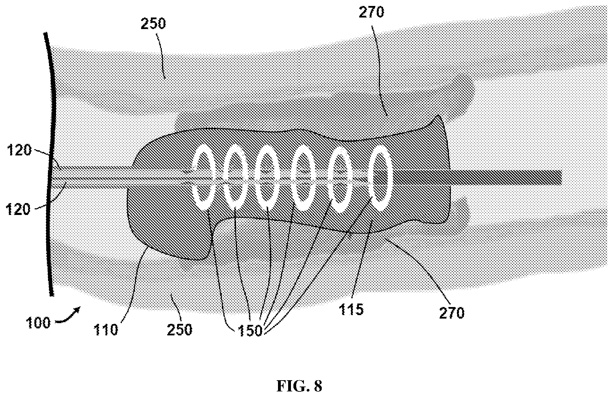

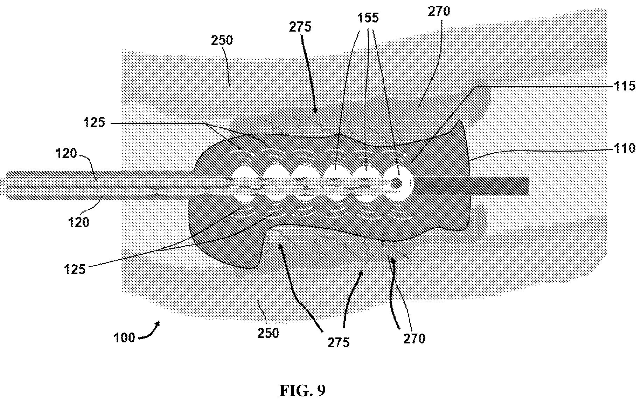

In FIG. 6, apparatus 100 has been inserted into artery 250 with calcium 270. It is understood that a guidewire (not shown) may be used for the deployment of this embodiment in a manner similar to the embodiment shown and described in FIGS. 1-4. In FIG. 7, pressurized fluid 115 has expanded expandable member 110 within artery 250 to conform to the contours of artery 250 and calcium 270. In FIG. 8, a laser light source (*e.g.* equivalent to light source 130 shown in FIG. 2) has been activated so that electromagnetic energy 150 is emitted from emission points 140. As shown in FIG. 9, electromagnetic energy 150 creates cavitation 155 (*e.g.* bubbles) in fluid 115 which generates ultrasonic waves 125 from the formation and collapse of the bubbles in fluid 115.

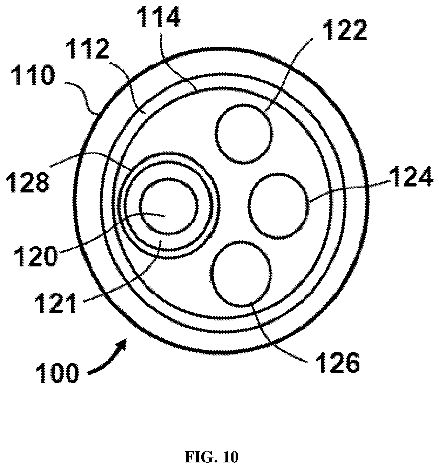

Referring now to FIG. 10 a schematic cross-sectional end view is shown of a specific embodiment that comprises additional ports, as discussed further below. FIG. 10 illustrates an embodiment of apparatus 100 comprising expandable member 110 coupled to a catheter 114 via a weld (*e.g.* an ultrasonic weld) 112. In the embodiment shown, apparatus 110 comprises fluid ports 122 and 124 configured to deliver fluid (*e.g.* a saline fluid) to expandable member 110, as well as a vent or drain port 126 configured to evacuate fluid, *e.g.* in order to reduce the cross-sectional diameter and volume of expandable member 110 prior to removing apparatus from the artery. In addition, port 126 can be configured to vent or remove bubbles from expandable member 110 after delivery of electromagnetic energy 150.

The accumulation of bubbles from the expandable element (balloon) after activation of the laser light source is difficult to control and their removal is critical. Accumulated single or multiple bubbles from a previous laser activation can redirect (unfocused delivery) electromagnetic energy on subsequent laser shots, which in turn can lead to complications such as damage to the vessel walls, etc.

The illustrated embodiment also comprises a port 128 configured to receive optical fiber 120. In the embodiment shown, optical fiber 120 is located within a conduit 121. In certain embodiments, conduit 121 may be configured as capillary tubing, and in a specific embodiments, conduit 121 is Polymicro Flexible Fused Silica Capillary Tubing with an inner diameter 200µm and an outer diameter of 350µm, available from Molex^{®}. Optical fiber 120 can provide imaging (including, for example, optical coherence tomography [OCT] imaging) of the procedure in real-time to provide visual feedback to the user of the extent of calcium fracture and allow for more precise control of apparatus 100.

In particular embodiments, OCT imaging may be use for other aspects in lieu of or in addition to calcium fracture detection. For example, in certain embodiments OCT imaging may be used for navigation, calcium plaque identification and estimation of the size to identify the treatment regimen (*e.g.* to provide more precise treatment), and laser control.

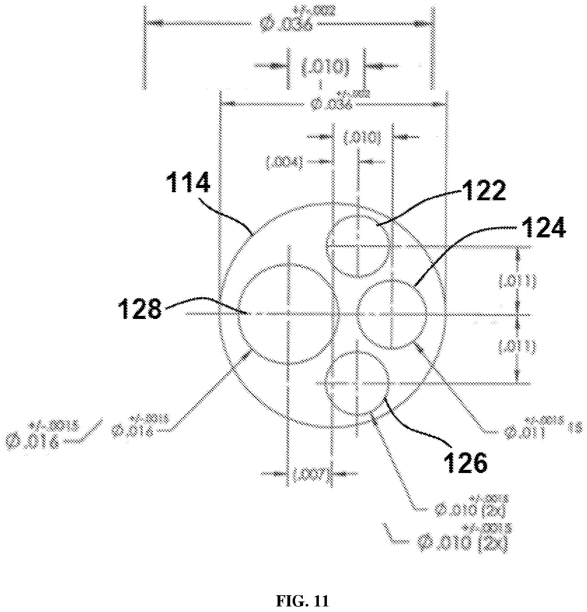

FIG. 11 illustrates an end dimensional view with dimensions for one specific embodiment of catheter 114 with fluid supply ports 122 and 124, a vent or drain port 126 and port 128 for an optical fiber. It is understood that other embodiments may comprise a configuration with different dimensions for the aspects shown in FIG. 11.

Exemplary embodiments of the present disclosure provide many benefits and advantages through the fracturing of intravascular calcium in the techniques disclosed herein. For example, the use of light (*e.g.* laser) energy has stark advantages in comparison to the use of electricity to generate the appropriate sonic waves. These advantages include a greater net energy delivered for a given form factor of a catheter device. In addition, exemplary embodiments of the present disclosure provide more control on the laser-water interaction through pulse duration, pulse repetition rate, wavelength, fluence/fluence rate. Furthermore, exemplary embodiments provide for beam shaping allowing for bubble formations that are conductive for a given desired sonic propagation pattern. In addition, exemplary embodiments may be provided for a more economical catheter given the price of an optical fiber. Further, the use of electricity can require pacing with each pulse, while there is no pacing of the heart with light.

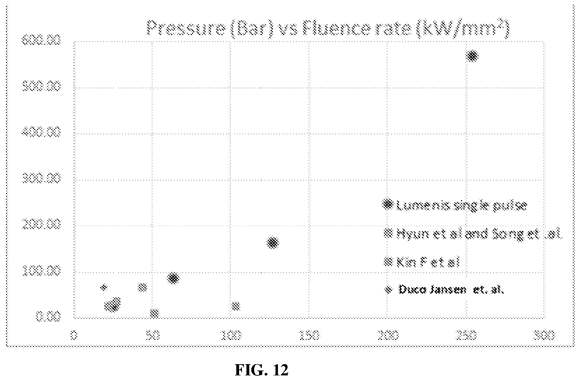

Utilizing electromagnetic *(*e.g. laser) energy to generate the sonic pressure within an expandable member (*e.g.* balloon), is believed to provide a more effective lithotripsy device for fracturing the arterial calcium in the vessel wall and increasing vessel compliance. Given the extremely high energy densities possible with fiber delivered laser pulses, ultrasonic pressures computed and/or measured are an order of magnitude higher than electrode generated pressure for a given form factor. As illustrated in FIG. 12, the peak pressure amplitude as a function of fluence rate shows values as high as 300 bars can be achieved delivering radiation with a 200 µm fiber. Comparatively, the maximum pressure amplitude reported in some of the studies by others (*e.g.* Shockwave Medical Inc., Santa Clara CA) ranges on the order of 40-50 bar. This suggests that the use of light allows for generation of multiple shock waves at a single time, or the fracture of larger collections of calcium such as calcium nodules.

The higher amplitude of the pressure waves generated during laser induced bubble formation and collapse could promote greater and more beneficial fracturing in the calcium. Triggering laser radiation also has the advantage of finer temporal control of the bubble creation that generates the pressure as compared to other techniques, including the use of electrode-generated electrical current. During testing of exemplary embodiments of the present disclosure, temporal videography of the laser generated bubbles shows a more uniform controlled formation with a laser as opposed to the electrically generated bubbles, possibly due to the higher levels of noise in electrical current and complex and sometimes chaotic thermomechanical-electrical interactions.

While other techniques have used imaging to verify efficacy after treatment, exemplary embodiments of the present disclosure can provide real-time imaging feedback on the procedure. Such feedback is needed to determine the laser dosimetry that would be required to increase vessel compliance in arteries with complicated calcification patterns. Exemplary embodiments of the present disclosure can couple high intensity light sources like (*e.g.* multi-photon, including two-photon light sources) with an imaging methodology into a single double clad fiber. Such a configuration highlights how optical coherence tomography (OCT) imaging could be incorporated into a catheter as a feedback during laser lithotripsy to assess the effects of treatment. Additionally, OCT could also guide in directing the treatment by detecting calcium in the arterial wall ensuring that the acoustics effects from the laser lithotripsy can be dialed-in based on the location and burden of calcium. In certain embodiments, OCT imaging can provide guidance not only by detecting calcified lesions or calcium plaque, but also by calcium scoring in real time using using measurements of parameters such as thickness, length and angle.Exemplary embodiments may include any of a number of choices for laser-water interactions. Water has absorption peaks at 1.3 µm, 1.94 µm, 2.07 µm, 2.94 µm. Corresponding readily available lasers at these wavelengths are neodymium yttrium aluminum garnet (Nd:YAG), Thulium (Tm), holmium yttrium aluminum garnet (Ho:YAG) and Erbium (Er:YAG).

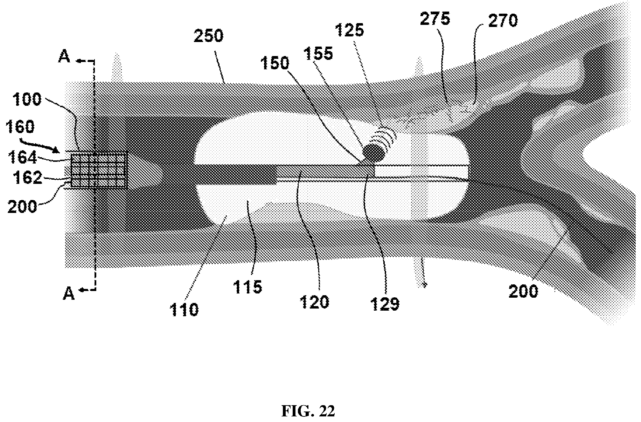

Referring now to FIG. 22, an overview of an exemplary apparatus and method of use are demonstrated. This embodiment is similar to previously-described embodiments, and also comprises one or more intravascular imaging devices. For purposes of clarity, not all features shown in each figure are labeled with reference numbers in every figure. For example, apparatus 100 may comprise a laser light source and a control system configured to control operational parameters of apparatus 100, including for example, the operation of the laser light source *(e.g.* laser pulse duration, frequency, amplitude *et al.*) similar to control system 135 and laser light source 130 shown in FIG. 3.

In FIG.22, a guide wire 200 has been inserted into a coronary artery 250 with calcium 270 located within artery 250. In this embodiment a portion of apparatus 100 has been inserted over guidewire 200 into artery 250. In the embodiment shown, apparatus 100 comprises an expandable member 110 (*e.g.* a balloon) and an optical fiber 120 coupled to a laser light source (*e.g.* equivalent to laser light source 130 in FIG. 3).

Apparatus 100 also comprises an intravascular imaging device 160. In the particular embodiment shown, intravascular imaging device 160 is configured as an intravascular ultrasound (IVUS) device comprising an ultrasonic transceiver 162 that comprises a plurality of transducers 164 extending around the perimeter of ultrasonic transceiver 162. In certain embodiments, transducers 164 are arranged circumferentially in one or more rows around ultrasonic transceiver 162. In exemplary embodiments, transducers 164 can be configured to provide imaging data from the entire interior circumference of the lumen (*e.g*. artery 250) into which ultrasonic transceiver 162 is inserted. In specific embodiments, ultrasonic transceiver 162 may incorporate aspects of commercially available systems, including for example, the Eagle Eye Platinum digital intravascular ultrasound (IVUS) available from Koninklijke Philips N.V^{®}.

Exemplary embodiments comprising transducers 164 extending around the perimeter of ultrasonic transceiver 162 can provide certain features not found in other embodiments, including for example, those incorporating a rotating array of transducers. For example, with guidewire 200 extending through the interior of ultrasonic transceiver 162, guidewire 200 does not produce artifacts because the photoacoustic signals are transmitted and received from multiple points around the circumference of transceiver 162. Accordingly, guidewire 200 does not block the transmission or reception of photoacoustic signals for each of transducers 164 extending around the perimeter of ultrasonic transceiver 162, and would not produce an artifact (in contrast a rotating linear array of transducers).

In addition, embodiments incorporating circumferential transducers 164 can transmit and receive photoacoustic signals from multiple points around the circumference of transceiver 162 without moving transceiver 162. Accordingly, transceiver 162 does not need to be rotated to provide imaging data for the interior circumference of artery 250. The ability to provide circumferential imaging data without rotating transceiver 162 can provide for a reduced diameter of apparatus 100 as compared to embodiments that require a mechanism to rotate an imaging device. Accordingly, apparatus 100 shown in FIG. 22 can be inserted into smaller diameter lumens, e.g. peripheral arteries as compared to coronary arteries.

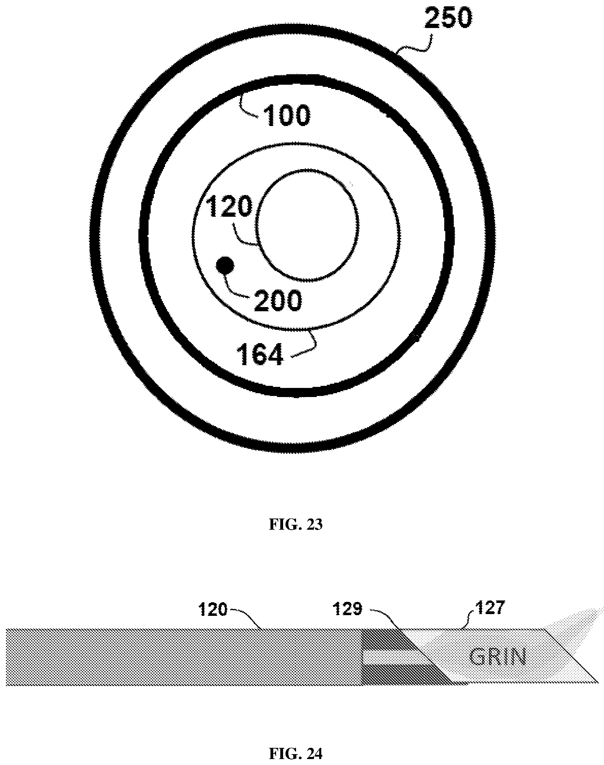

In the embodiment shown in FIG. 23 (a section view taken along line A-A in FIG. 22), apparatus 100 has an outer diameter of approximately 1.5 millimeters (mm). Transceiver 162 has an outer diameter of approximately 1.2 mm, optical fiber 120 has an outer diameter of approximately 0.32 mm, and guidewire 200 has an outer diameter of approximately 0.23 mm. Both guidewire 200 and optical fiber 120 extend through transceiver 162, which is located within the 1.5 mm diameter catheter of apparatus 100. It is understood that the diameters disclosed herein are merely exemplary of one embodiment, and other embodiments may comprise components with different diameters. While not shown for purposes of clarity, it is understood that the embodiment shown in FIGS. 22-23 may also comprise one or more fluid ports configured to deliver fluid to expandable member 110, as well as a vent or drain port configured to evacuate fluid from expandable member 110 equivalent to those in previously described embodiments.

In the embodiment shown in FIG. 22, expandable member 110 has been expanded within artery 250 via a fluid 115 that is pressurized within expandable member 110. **In** particular embodiments of the present disclosure fluid 115 may be saline, or indocyanine green (ICG), an FDA approved solution resulting in an absorption coefficient more than five times greater than saline. It is understood that other embodiments disclosed herein may comprise saline or ICG as well.

In this embodiment, optical fiber 120 extends through transceiver 162 and into the interior of expandable member 110. During operation, optical fiber 120 can transmit electromagnetic energy 150 from a distal end 129. In particular embodiments, distal end 129 is configured to transmit electromagnetic energy 150 in a particular direction toward artery 250. For example, distal end 129 may be configured (*e.g.* beveled, tapered, faceted or angled) to provide directional transmission of electromagnetic energy 150. By utilizing intravascular imaging device 160 to determine the location of calcium 270 within artery 250, a user can direct or target electromagnetic energy 150 toward calcium 270. In certain embodiments, electromagnetic energy 150 is provided by a diode-laser (793 nm, 0.6kW available from DILAS Coherent^{®} Inc.). A 793 nm wavelength is suitable for an inflatable member filled with ICG fluid, which provides strong optical absorption in the 790-810 nm range.

As previously discussed, electromagnetic energy 150 creates cavitation 155 (*e.g.* bubbles) in fluid 115 which generates ultrasonic waves 125 from the formation and collapse of the bubbles 155 in fluid 115. By directing electromagnetic energy 150 toward calcium 270, cavitation 155 and ultrasonic waves 125 are also directed toward calcium 270 and not toward portions of artery 250 where calcium 270 is not deposited. Accordingly, portions of artery 250 that do not include deposits of calcium 270 are not subjected to the forces associated with cavitation 155 and ultrasonic waves 125, and are therefore less likely to be damaged by such forces. Because calcium deposits 270 are not uniformly distributed, the ability to obtain imaging data of vessel 250 to determine the locations of calcium 270 and target electromagnetic energy 150 to such locations can provide for increased safety and reduced risks to patients.

Certain embodiments may also incorporate other mechanisms for obtaining imaging data within artery 250. For example, referring now to FIG. 24, in certain embodiments optical fiber 120 may be configured as a double clad fiber (*e.g.* a DCF13 fiber available from Thorlabs^{©} Inc.) with a gradient-index (GRIN) lens 127 coupled to distal end 129. In such embodiments, GRIN lens 127 can be used for obtaining optical coherence tomography (OCT) image data beyond distal end 129.

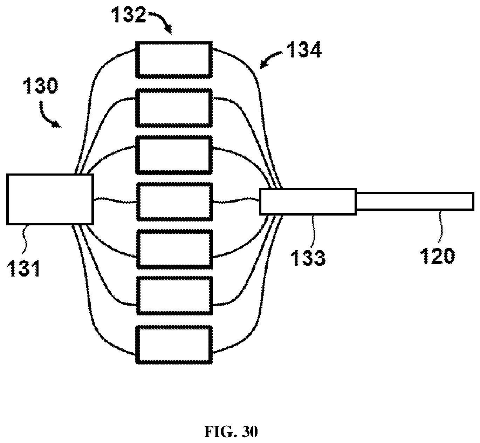

Referring now to FIG. 30, one embodiment of laser light source 130 is shown comprising a power source 131 electrically coupled to a plurality of diode lasers 132. In the embodiment shown, diode lasers 132 are coupled to optical fiber 120 via a fiber combiner 133 and optical fibers 134. In particular embodiments, diode lasers 132 may be 793 nm or 808 nm lasers emitting 100 watts, which emit electromagnetic energy at a wavelength near the maximum absorption coefficient for a specified concentration of an ICG formulation in the expandable member (not shown in FIG. 30) coupled to optical fiber 120. In particular embodiments, optical fibers 134 may be 105 µm or 125 µm silica core fibers, and optical fiber 134 may be a biocompatible 250 µm fiber.

This embodiment can provide the higher levels of electromagnetic pulsed energy coupled with an absorbing fluid medium at lower cost by combining multiple diode lasers with one power supply and fiber combiner. In particular embodiments, nineteen diode lasers may be coupled to one power supply, but other embodiments may comprise a different number of diode lasers. The use of diode lasers also provides for a compact configuration and flexible pulse profile. Accordingly, embodiments utilizing multiple diode lasers can provide sufficient electromagnetic energy to an absorbing biocompatible fluid in an expandable member to effectively fracture calcium.

In addition, the absorbing biocompatible fluid in the expandable member can be configured to efficiently fracture calcium with respect to the electromagnetic energy provided. As molar concentration of ICG increases in solution, the absorption coefficient also increases. However, this increase is not linear. Hence, if 1x concentration is 1cm⁻¹, 100x is not necessarily 100cm⁻¹. This is because of an "aggregation" effect of cyanine dyes. Cyanine dyes, including ICG, tend to aggregate at high concentration in aqueous solutions, which can reduce the absorption coefficient.

A lower aggregation implies lower power needed to generate the same pressure. While dimethyl sulfoxide (DMSO) can be used to avoid aggregation in ex vivo applications, it is not biocompatible. Accordingly exemplary embodiments of the present disclosure can comprise other techniques, including for example, dissolving the dye in liposome-type nano droplets. In addition, exemplary embodiments of the present disclosure can utilize plasma or albumin instead of water in the solution to increase the absorption coefficient.

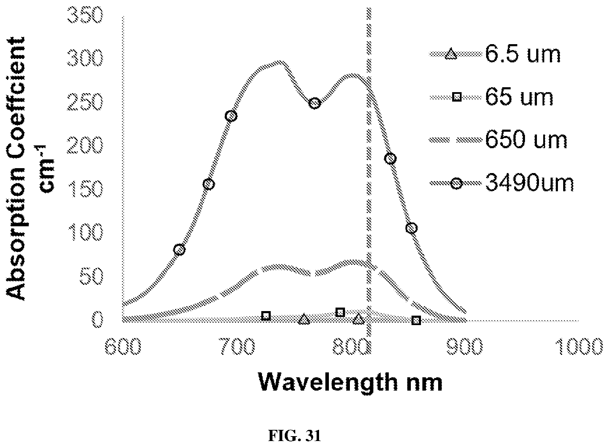

Referring now to FIG. 31, the absorption coefficient of ICG versus wavelength is shown for different concentrations of ICG in a saline water solution. ICG has an absorption coefficient of >256cm⁻¹ at 808 nm, and the peak power requirement drops by a factor of 5X with a reduction in aggregation (*e.g.,* a 5X cost reduction).

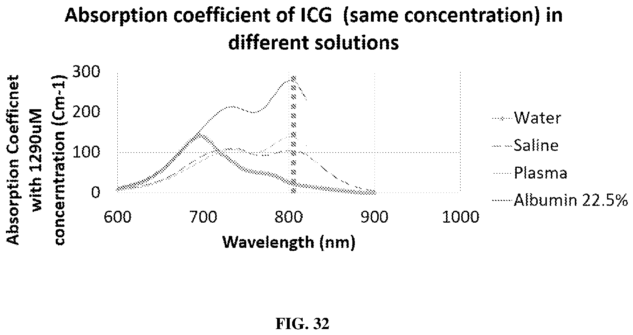

The absorption coefficient of ICG is also affected by the solution in which the ICG is diluted. Referring now to FIG. 32, a graph is shown of absorption coefficient versus wavelength for the same concentration of ICG in different solutions. As shown in FIG. 32, albumin provided the highest absorption coefficient, while water had the lowest. An excimer wavelength of 308 nm has an absorption coefficient of about 100cm⁻¹ in serum albumin. With ICG mixed in with albumin, the absorption coefficient is higher, and with an iodine contrast (such as those used in x-ray fluoroscopy or x-ray angiography mixed with saline, *e.g.* OmniPaqueTM (iohexol), ioversol *etc*.) at a 50/50 percent mixture, the absorption coefficient is about 400-500cm⁻¹.

Pure or 100% contrast results in an absorption coefficient of 900-1000cm⁻¹, but it is difficult to flow contrast through tiny lumens to fill intravascular balloons as 100% contrast is sticky and very viscous in small lumens. However, if the contrast is mixed with 50/50 percent water or saline, it flows easier. This mixture provides easy flow to fill a balloon and cause shockwave generation needed to fracture the calcium. Additionally, if the contrast is mixed with blood or hemoglobin, the pressures from shockwave are seen to be higher while keeping the flow consistent to fill intravascular balloons.

In summary, testing indicates an expandable member (*e.g*. balloon) filled with 100 percent contrast can achieve pressures of about 50 bars of pressure with an excimer wavelength of 308 nm. A mixture of blood/hemoglobin in the balloon and an excimer wavelength of 308 nm can also reach pressures of 50 bars. Accordingly, one can utilize contrast in a balloon and illuminate the solution at excimer wavelength of 308 nm to achieve sufficient pressure amplitudes to cause calcium fracture.

In summary, testing indicates an expandable member (*e.g.* balloon) filled with 100 percent contrast can achieve pressures of about 50 bars of pressure with an excimer wavelength of 308 nm. A mixture of blood/hemoglobin in the balloon and an excimer wavelength of 308 nm can also reach pressures of 50 bars. Accordingly, one can utilize contrast in a balloon and illuminate the solution at excimer wavelength of 308 nm to achieve enough pressure to cause calcium fracture.

### RESULTS

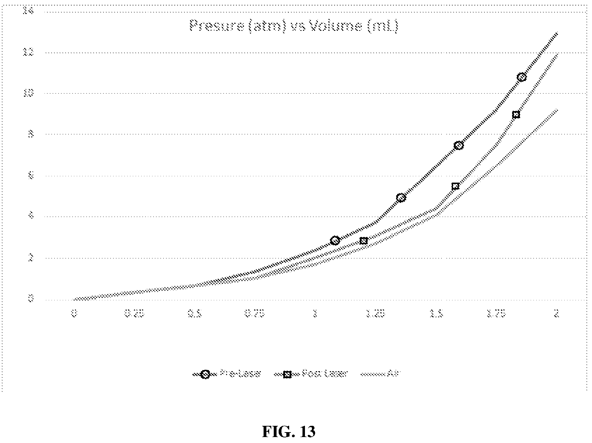

FIGS. 13-16 illustrate results of testing as further described below. For this testing, a Ho:YAG laser at a wavelength of 2.07 µm was selected with a pulse duration of about 150ms. Given what is known about the laser-water interaction, the best choice of a laser dosimetry would be one with a shorter pulse duration (ns), high water absorption and high energy density laser modules. That would suggest Er:YAG (which has the higher water absorption coefficient at 2.94um). Er:YAG delivery fibers like germanium are not biocompatible to implement in one of these catheters. An optimum choice would thus be a thulium (1.94 um) nanosecond pulse duration laser that can deliver between 1 uJ to 5J of pulsed laser energy. However, given relative availability of higher energy laser pulses at this wavelength, the closest choice of Ho:YAG was utilized for this testing.

To test the ability of lasers to generate calcium fracturing pressure waves a pilot study was conducted in n=9 freshly harvest human coronary arteries which were calcified. The arterial compliance was measured before and after treatment with a holmium laser, as well as performing OCT imaging and histology.

Hearts were received from South Texas Blood and Tissue. The inclusion criteria for hearts was a history of CAD or factors indicative of CAD and calcium burden, *i.e.* older age, excessive body weight, hypertension, previous bypass surgery, and diabetes mellitus. Coronary arteries were dissected from the heart. The left anterior descending (LAD), right coronary artery (RCA), and left circumflex (LCX) were all imaged with OCT. OCT was used to identify calcium in the vessel. Dye was used on the outside of the vessel to mark calcium location so that compliance testing and laser treatment could be targeted in the same area where calcium was present.

Following location identification, vessel compliance was measured. A balloon catheter was chosen based on the size of the vessel. A vessel compliance curve was obtained by using a manual balloon catheter pump (Endoflator^{®}), to inflate the balloon and recording the pressure of the balloon at given volumes of saline added. This curve was repeated 3 times at each of 4 conditions: in air before and after the other tests to measure the baseline compliance of the balloon and ensure that it did not vary during the experiment due to balloon fatigue; in the vessel before and after the laser treatment. The in-vessel balloon location was determined by the dye indicated calcium location.

For this testing, access to two holmium lasers, MOSES^{tm} Pulse 120H (Lumenis^{®}, Yokneam Israel) and a Coherent Holmium:YAG (Lumenis^{®}, Yokneam Israel) were available. These provided the energy source for the treatment through a conical tipped optical fiber. A variety of pulse numbers and patterns are tested on both lasers to determine optimal treatment options. These lasers differ 10-fold in the amount pulse energy they can deliver. An aiming beam on the laser allowed for the treatment to be directed to an area marked with dye. Following laser treatment a second vessel compliance measurement, and a follow-up OCT image were recorded. This second OCT image was then co-registered with the pre-test OCT image. The OCT images were analyzed for visible signs of calcium fracture and change in lumen area can be calculated for quantitative characterization. The delta of the compliance curves or increase in compliance before and after laser treatment was an endpoint measure for procedural success.

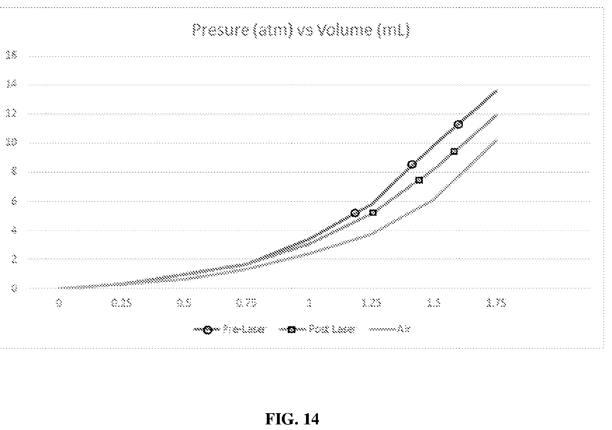

Nine coronary arteries from four human hearts have been tested. In each coronary artery a procedure success has been achieved with increased arterial compliance after laser treatment. FIGS. 13 and 14 illustrate graphs of recorded arterial compliance curves. Post-laser compliance (square markers) show improvement over pre-laser compliance (circle markers) while being higher than the balloons native compliance (solid line). If the post laser compliance were identical to the balloon in air laser compliance, then the coronary artery has an extremely large compliance suggesting damage to the arterial wall may have occurred.

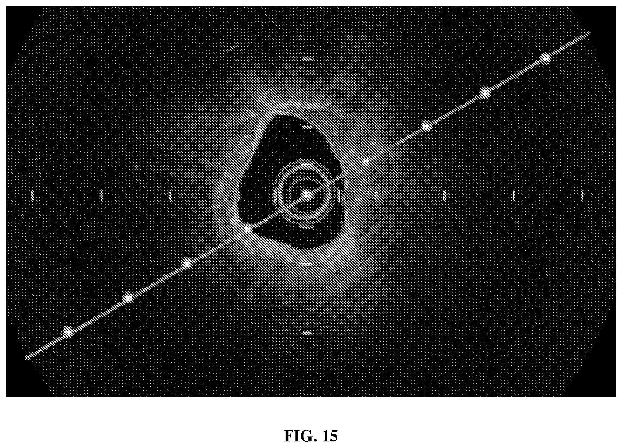

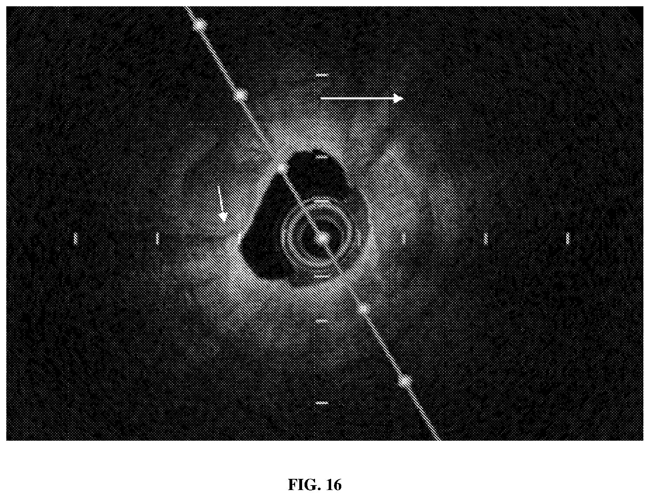

FIGS. 15 and 16 are optical coherence tomography (OCT) images of an artery containing calcium before (FIG. 15) and after (FIG. 16) treatment via the methods disclosed herein. As shown by the white arrows in FIG. 15, the calcium in the artery is fractured after treatment.

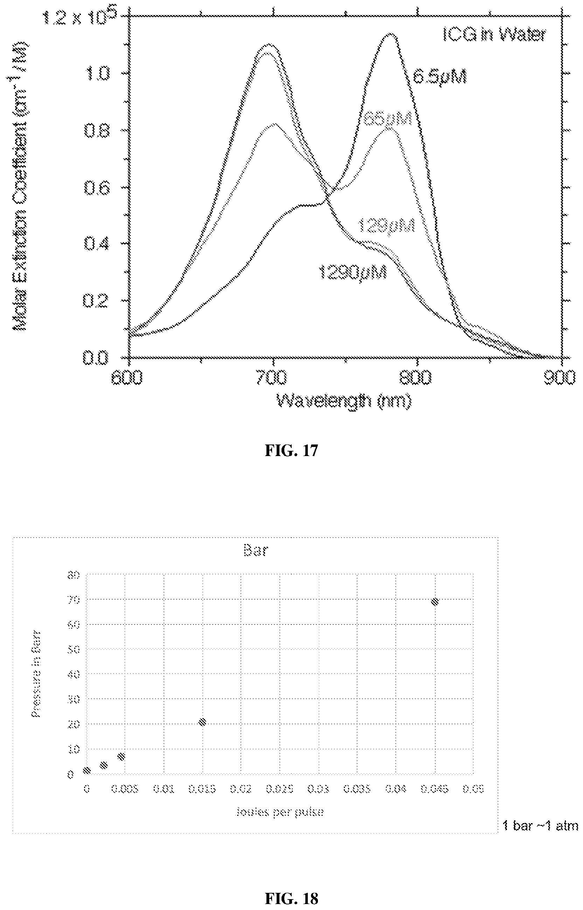

Referring now to FIGS. 17 and 18, data from an exemplary embodiment is shown with laser sources emitting radiation in 700-850nm wavelength range and is absorbed by indocyanine green (ICG). In this embodiment, ICG has an absorption spectrum between 700-850nm with absorption peaks tunable with concentration of ICG measured in micromolars (*see* e.g. https://omlc.org/spectra/icg/). For example, at 2.2mg/mL (maximum concentration in liquid form, 2830uM), the absorption coefficient can be as high as 240cm^-1 at 755nm, 311cm^-1 at 700nm. Compared to saline/water at wavelengths of absorption for holmium lasers this is a substantially higher value. *(e.g.* in comparison, the local maximum of 119.83cm-1 at water absorption peak of 1940nm Tm lasers and ~30cm-1 at 2.09-2.10 um Holmium lasers).

Use of an alternative fluid (to saline) for laser shock wave generation allows for use of existing lasers at approximately 755nm, including for example: Picosure (755nm, 900ps, 200mJ, manufactured by Cynosure); GentleLase: (755nm, >1ms, 25J, manufactured by Candela); Alexandrite: (750nm, 5-10ns, 150mJ); Laser Diode: (793nm, 1600W power, pulse duration: 100ns-100us, 500us-CW, other options 808nm, 1600W)

Shock wave pressure amplitudes recorded were as high as 1000psi (200mJ, 900ps). FIG. 18 provides a graph of pressure vs energy per pulse generated with ICG (~2.2mg/mL) with a Picosure laser manufactured by Cynosure (755nm, 900ps).

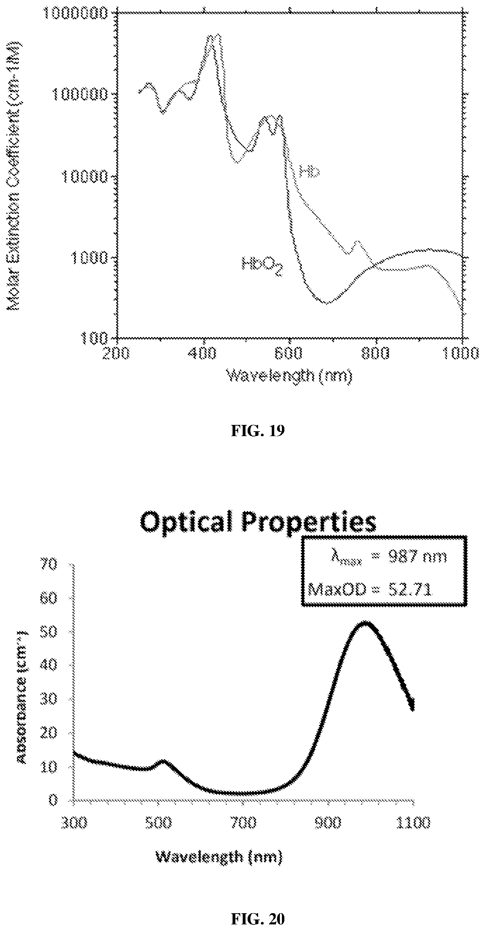

Referring now to FIG. 19, data from another embodiment was obtained using 500-600nm wavelength range laser sources with blood/Hb fluid solution contained in the balloon. Blood has an absorption peak at 532nm with a strength of about 250cm-1. The absorption of blood at 532nm is many times higher than water at the holmium laser emission wavelength (~30cm^-1). A candidate fluid to fill in the balloon could be biocompatible hemoglobin or whole blood from the same patient to generate required pressures to fracture calcium in the vessel wall.

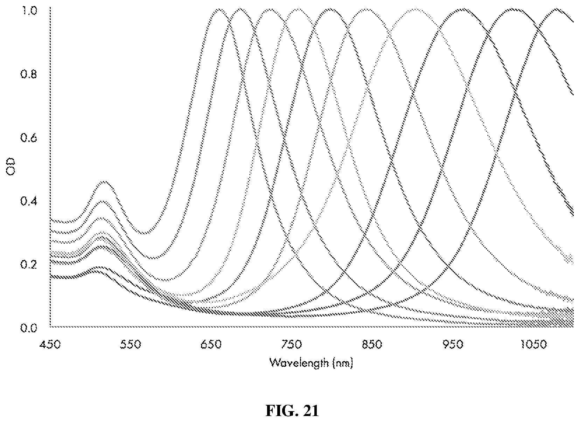

FIGS. 20 and 21, data from an embodiment comprising biocompatible nanoparticle solution inside the balloon is shown. In this embodiment, gold nanorods provide tunable absorption spectra. For example, nanorods produced by NanocomposiX and other manufacturers have a 980nm wavelength absorption peak (up to 100 optical density [OD], 230cm^-1). There are also numerous diode laser suppliers at 980nm (up to 570W delivered in a 100um silica fiber). Other biocompatible nanorods/nanoparticles are manufacturable and may be selected depending on availability of laser sources (808nm, 793nm, 980nm, 976nm, 1210nm, etc.) and corresponding optical fiber delivery options.

It is also noted, albumin (human serum albumin) when mixed with ICG or by itself has strong absorption at wavelengths in the ultraviolet (UV) spectrum. In certain embodiments, UV lasers (*e.g.* Xenon monochloride [XeCL]) excimer or other UV laser diodes can be utilized to generate shock waves in these albumin or albumin and ICG-filled balloons to fracture calcium in the vessel wall.

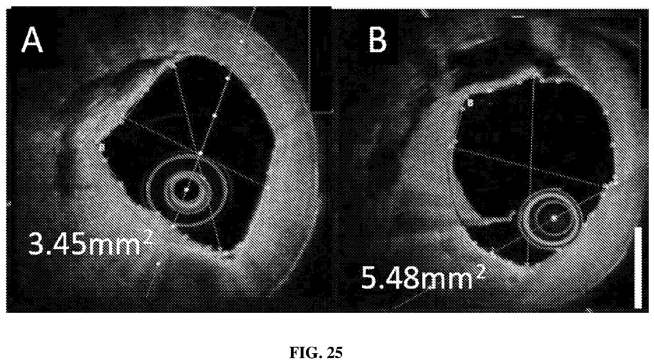

Referring now to FIG. 25, an OCT image of an ex vivo human artery is shown in panel A before undergoing laser induced lithotripsy procedures according to the present disclosure. FIG. 25 panel B shows and OCT image of the artery after laser induced lithotripsy was performed. As shown in panel B, fractures are formed in the calcium and the cross-sectional area of the artery is increased to 5.48 mm² (up from 3.45 mm² prior to laser induced lithotripsy).

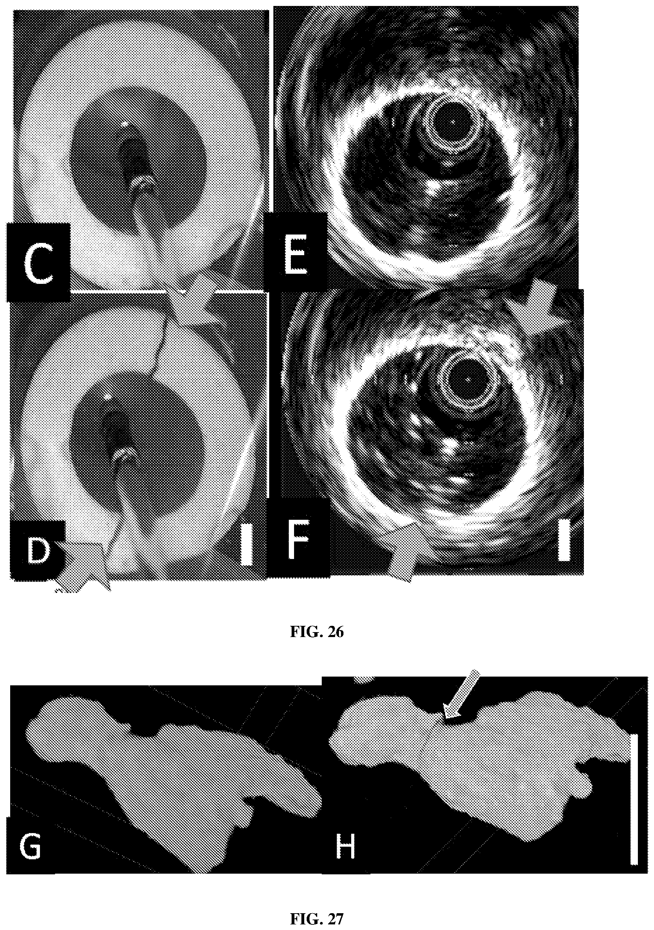

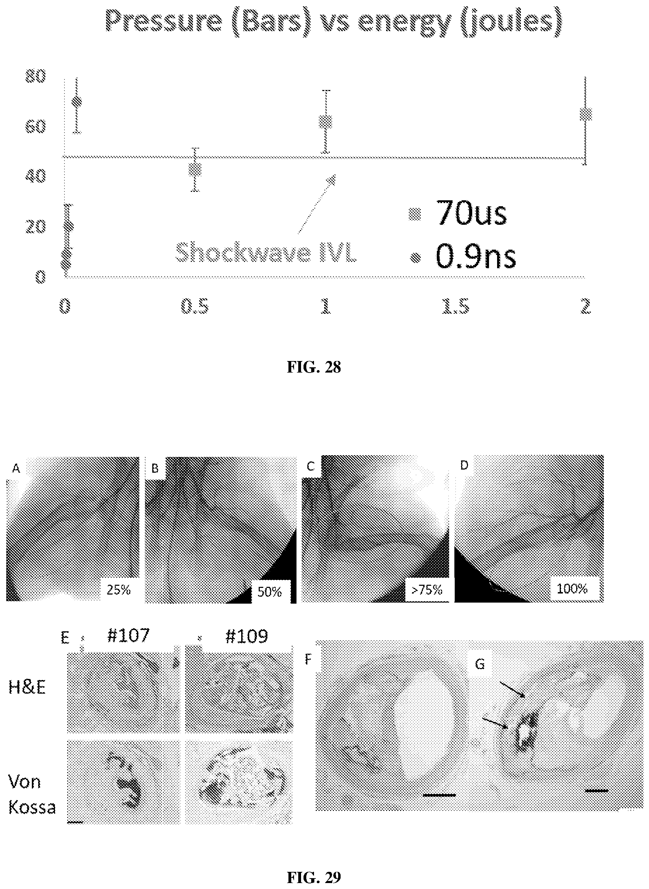

FIG. 26 panels C and D show images of Ultracal^{®} 30 stone before and after, respectively, of fractures demonstrated using a flat 230 um core fiber under IVUS guidance according to the present disclosure. FIG. 26 panels E and F show before and after IVUS imaging of laser induced lithotripsy fractures in calcified coronary phantoms (fractures indicated by arrows in panels D and F). FIG. 27 panels G and H show before and after micro-CT images of a human ex vivo artery demonstrating laser induced fracture (fracture indicated by arrow in panel H). FIG. 28 shows a graph of pressure (bars) versus energy (joules) for different pulse durations in ICG (circles) and saline (squares) with a 0.9ns and a 70us pulse duration delivered in a fiber. Scale bars are 1mm.

FIG. 29 panels A-D shows x-ray fluoroscopy of an in vivo rabbit model showing varying levels of stenosis from 25 percent to 100 percent . FIG. 29 panel E shows hematoxylin and eosin (H&E) and von Kossa staining in the top and bottom rows, respectively, of the model arteries at 4x magnification. The brown regions in the von Kossa stain are calcium. FIG. 29 panels F and G show laser induced shockwave fractures (black arrows in panel G) in ex vivo human arteries compared to controls (shown in panel F), with a scale bar of 1 mm.

All of the devices, systems and/or methods disclosed and claimed herein can be made and executed without undue experimentation in light of the present disclosure. While the devices and systems have been described in terms of particular embodiments, it will be apparent to those of skill in the art that variations may be applied to the devices, systems and/or methods in the steps or in the sequence of steps of the method described herein without departing from the scope of the invention. All such similar substitutes and modifications apparent to those skilled in the art are deemed to be within the scope of the invention as defined by the appended claims.

### REFERENCES:

1. Rocha-Singh et al, Peripheral arterial calcification: prevalence, mechanism, detection, and clinical implications, Catheter Cardiovasc Intervention, 2014
2. Ali et al, Optical Coherence Tomography Characterization of Coronary Lithoplasty for Treatment of Calcified Lesions, JACC Imaging, 2017
3. Warisawa et al, Successful Disruption of Massive Calcified Nodules Using Novel Shockwave Intravascular Lithotripsy, Circ J, 2020
4. Brinton et al, Feasibility of Shockwave Coronary Intravascular Lithotripsy for the Treatment of Calcified Coronary Stenoses, Circ J, 2019
   U.S. Patent 8,728,091
   U.S. Patent 8,747,416
   U.S. Patent 8,888,788
   U.S. Patent 8,956,371
   U.S. Patent 8,956,374
   U.S. Patent 9,005,216
   U.S. Patent 9,011,462
   U.S. Patent 9,011,463
   U.S. Patent 9,072,534
   U.S. Patent 9,138,249
   U.S. Patent 9,333,000
   U.S. Patent 9,433,428
   U.S. Patent 9,642,673
   U.S. Patent 10,206,698
   U.S. Patent 10,039,561
   U.S. Patent 10,159,505
   U.S. Patent 10,517,621
   U.S. Patent 10,682,178
   U.S. Patent 10,702,293
   U.S. Patent 10,709,462

## Claims

1. An apparatus (100) configured to fracture coronary calcium (270), the apparatus comprising:
an expandable member (110);
a laser light source (130); and
an optical fiber (120) coupled to the laser light source (130), wherein:
the expandable member (110) is a balloon;
the laser light source (130) comprises a diode laser;
the optical fiber (120) comprises emission points (140) configured to emit electromagnetic energy (150) from the laser light source (130) from the optical fiber; and
emission of electromagnetic energy (150) from the emission points (140) is configured to create fractures (275) in the coronary calcium.

2. The apparatus of claim 1 wherein:
the expandable member (110) comprises a fluid (115); and
the emission of electromagnetic energy (150) from the emission points (140) is configured to create fractures (275) in the coronary calcium (270) by generating ultrasonic waves (125) in the fluid.

3. The apparatus of any one of the preceding claims wherein the emission points (140) are configured as conical reliefs in the optical fiber (120).

4. The apparatus of any one of the preceding claims wherein:
the optical fiber (120) is a first optical fiber;
the apparatus (100) further comprises a plurality of optical fibers (134); and
each optical fiber of the plurality of optical fibers comprises emission points (140) configured to emit electromagnetic energy (150) in a radial pattern from each optical fiber.

5. The apparatus of any one of the preceding claims wherein the expandable member (110) is configured to be expanded via a fluid (115) contained within the expandable member,
preferably wherein the apparatus (100) further comprises a first port (122, 124) configured to deliver the fluid to the expandable member,
more preferably wherein the apparatus further comprises a second port (126) configured to drain the fluid from the expandable member, preferably wherein the second port is further configured to evacuate vapor bubbles from the expandable member.

6. The apparatus of claim 5 wherein the fluid (115) is configured to absorb electromagnetic energy (150) from the optical fiber (120), generate an acoustic wave and propagate to the calcium (270).

7. The apparatus of claim 5 or 6 wherein the fluid (115) is a saline fluid.

8. The apparatus of any one of the preceding claims wherein the optical fiber (120) is configured to emit the electromagnetic energy (150) in a radial pattern.

9. The apparatus of any one of the preceding claims wherein the electromagnetic energy (150) is emitted at a wavelength of approximately 2 µm.

10. The apparatus of any one of the preceding claims wherein the electromagnetic energy (120) is emitted at a wavelength between 1.5 µm and 2.5 µm.

11. The apparatus of any one of the preceding claims further comprising an intravascular imaging device (160).

12. The apparatus of claim 11 wherein the intravascular imaging device (160) is an intravascular ultrasound (IVUS) device, or wherein the intravascular imaging device is an optical coherence tomography imaging (OCT) device.

13. The apparatus of any one of claims 1 to 12, wherein:
the optical fiber (120) comprises a proximal end and a distal end (129); and
the optical fiber is configured to emit electromagnetic energy (150) from the laser light source (130) from the distal end of the optical fiber.

14. The apparatus of any one of claims 2-13 wherein the fluid (115) comprises indocyanine green (ICG) and/or the fluid absorbs radiation emitted by the laser light source (130).

15. The apparatus of claim 13 or 14 wherein the laser light source (130) is configured to emit electromagnetic energy (150) at an emission wavelength; and
the fluid (115) has an absorption coefficient at the emission wavelength; and/or
wherein the emission of electromagnetic energy (150) is configured to allow flexible temporal and spatial control over shock-wave generation.

## Patentansprüche

1. Vorrichtung (100), die zum Aufbrechen von Koronarkalk (270) konfiguriert ist, wobei die Vorrichtung aufweist:
ein expandierbares Element (110);
eine Laserlichtquelle (130); und
eine optische Faser (120), die mit der Laserlichtquelle (130) gekoppelt ist, wobei:
das expandierbare Element (110) ein Ballon ist;
die Laserlichtquelle (130) einen Diodenlaser aufweist;
die optische Faser (120) Emissionspunkte (140) aufweist, die konfiguriert sind, elektromagnetische Energie (150) von der Laserlichtquelle (130) aus der optischen Faser zu emittieren; und
eine Emission elektromagnetischer Energie (150) aus den Emissionspunkten (140) konfiguriert ist, Frakturen (275) im Koronarkalk zu erzeugen.

2. Vorrichtung nach Anspruch 1, wobei:
das expandierbare Element (110) ein Fluid (115) aufweist; und
die Emission elektromagnetischer Energie (150) aus den Emissionspunkten (140) konfiguriert ist, Frakturen (275) im Koronarkalk (270) durch Erzeugen von Ultraschallwellen (125) in dem Fluid zu erzeugen.

3. Vorrichtung nach einem der vorhergehenden Ansprüche, wobei die Emissionspunkte (140) als konische Reliefs in der optischen Faser (120) konfiguriert sind.

4. Vorrichtung nach einem der vorhergehenden Ansprüche, wobei:
die optische Faser (120) eine erste optische Faser ist;
die Vorrichtung (100) ferner mehrere optische Fasern (134) aufweist; und
jede optische Faser der mehreren optischen Fasern Emissionspunkte (140) aufweist, die konfiguriert sind, elektromagnetische Energie (150) in einem radialen Muster aus jeder optischen Faser zu emittieren.

5. Vorrichtung nach einem der vorhergehenden Ansprüche, wobei das expandierbare Element (110) konfiguriert ist, durch ein Fluid (115) expandiert zu werden, das in dem expandierbaren Element enthalten ist,
wobei die Vorrichtung (100) vorzugsweise ferner eine erste Öffnung (122, 124) aufweist, die konfiguriert ist, das Fluid dem expandierbaren Element zuzuführen,
wobei die Vorrichtung besonders bevorzugt ferner eine zweite Öffnung (126) aufweist, die konfiguriert ist, das Fluid aus dem expandierbaren Element abzulassen, wobei die zweite Öffnung vorzugsweise ferner konfiguriert ist, Dampfblasen aus dem expandierbaren Element zu evakuieren.

6. Vorrichtung nach Anspruch 5, wobei das Fluid (115) konfiguriert ist, elektromagnetische Energie (150) von der optischen Faser (120) zu absorbieren, eine Schallwelle zu erzeugen und diese zum Kalk (270) auszubreiten.

7. Vorrichtung nach Anspruch 5 oder 6, wobei das Fluid (115) ein salzhaltiges Fluid ist.

8. Vorrichtung nach einem der vorhergehenden Ansprüche, wobei die optische Faser (120) konfiguriert ist, die elektromagnetische Energie (150) in einem radialen Muster zu emittieren.

9. Vorrichtung nach einem der vorhergehenden Ansprüche, wobei die elektromagnetische Energie (150) bei einer Wellenlänge von etwa 2 µm emittiert wird.

10. Vorrichtung nach einem der vorhergehenden Ansprüche, wobei die elektromagnetische Energie (120) bei einer Wellenlänge zwischen 1,5 µm und 2,5 µm emittiert wird.

11. Vorrichtung nach einem der vorhergehenden Ansprüche, die ferner eine intravaskuläre Bildgebungsvorrichtung (160) aufweist.

12. Vorrichtung nach Anspruch 11, wobei die intravaskuläre Bildgebungsvorrichtung (160) eine intravaskuläre Ultraschall- (IVUS) Vorrichtung ist oder wobei die intravaskuläre Bildgebungsvorrichtung eine optische Kohärenztomographie-Bildgebungs- (OCT) Vorrichtung ist.

13. Vorrichtung nach einem der Ansprüche 1 bis 12, wobei:
die optische Faser (120) ein proximales Ende und ein distales Ende (129) aufweist; und
die optische Faser konfiguriert ist, elektromagnetische Energie (150) von der Laserlichtquelle (130) aus dem distalen Ende der optischen Faser zu emittieren.

14. Vorrichtung nach einem der Ansprüche 2 bis 13, wobei das Fluid (115) Indocyaningrün (ICG) aufweist und/oder das Fluid von der Laserlichtquelle (130) emittierte Strahlung absorbiert.

15. Vorrichtung nach Anspruch 13 oder 14, wobei die Laserlichtquelle (130) konfiguriert ist, elektromagnetische Energie (150) bei einer Emissionswellenlänge zu emittieren; und
das Fluid (115) einen Absorptionskoeffizienten bei der Emissionswellenlänge aufweist; und/oder
wobei die Emission elektromagnetischer Energie (150) konfiguriert ist, eine flexible zeitliche und räumliche Steuerung der Stoßwellenerzeugung zu ermöglichen.

## Revendications

1. Un appareil (100) configuré pour fracturer le calcium coronaire (270), l'appareil comprenant:
un élément expansible (110) ;
une source de lumière laser (130) ; et
une fibre optique (120) couplée à la source de lumière laser (130), dans lequel :
l'élément expansible (110) est un ballonnet ;
la source de lumière laser (130) comprend un laser à diode ;
la fibre optique (120) comprend des points d'émission (140) configurés pour émettre
de l'énergie électromagnétique (150) depuis la source de lumière laser (130) à partir de la fibre optique ; et
l'émission d'énergie électromagnétique (150) depuis les points d'émission (140) est configurée pour créer des fractures (275) dans le calcium coronaire.

2. L' appareil selon la revendication 1, dans lequel :
l'élément expansible (110) comprend un fluide (115) ; et
l'émission d'énergie électromagnétique (150) depuis les points d'émission (140) est configurée
pour créer des fractures (275) dans le calcium coronaire (270) en générant des ondes ultrasonores (125) dans le fluide.

3. L' appareil selon l'une quelconque des revendications précédentes, dans lequel les points d'émission (140) sont configurés sous la forme de reliefs coniques dans la fibre optique (120).

4. L' appareil selon l'une quelconque des revendications précédentes, dans lequel :
la fibre optique (120) est une première fibre optique ;
l'appareil (100) comprend en outre une pluralité de fibres optiques (134) ; et
chaque fibre optique de la pluralité de fibres optiques comprend des points d'émission (140)
configurés pour émettre de l'énergie électromagnétique (150) selon un motif radial depuis chaque fibre optique.

5. L' appareil selon l'une quelconque des revendications précédentes, dans lequel l'élément expansible (110) est configuré pour être expansé au moyen d'un fluide (115) contenu à l'intérieur de l'élément expansible,
de préférence dans lequel l'appareil (100) comprend en outre un premier orifice (122, 124) configuré pour délivrer le fluide à l'élément expansible,
plus préférablement dans lequel l'appareil comprend en outre un second orifice (126) configuré pour drainer le fluide de l'élément expansible, de préférence dans lequel le second orifice est en outre configuré pour évacuer les bulles de vapeur de l'élément expansible.

6. L'appareil selon la revendication 5, dans lequel le liquide (115) est configuré pour absorber l'énergie électromagnétique (150) provenant de la fibre optique (120), générer une onde acoustique et la propager jusqu'au calcium (270).

7. L'appareil selon la revendication 5 ou 6, dans lequel le liquide (115) est un liquide salin.

8. L'appareil selon l'une quelconque des revendications précédentes, dans lequel la fibre optique (120) est configurée pour émettre l'énergie électromagnétique (150) selon un motif radial.

9. L'appareil selon l'une quelconque des revendications précédentes, dans lequel l'énergie électromagnétique (150) est émise à une longueur d'onde d'environ 2 µm.

10. L'appareil selon l'une quelconque des revendications précédentes, dans lequel l'énergie électromagnétique (120) est émise à une longueur d'onde comprise entre 1,5 µm et 2,5 µm.

11. L'appareil selon l'une quelconque des revendications précédentes, comprenant en outre un dispositif d'imagerie intravasculaire (160).

12. L'appareil selon la revendication 11, dans lequel le dispositif d'imagerie intravasculaire (160) est un dispositif d'échographie intravasculaire (IVUS), ou dans lequel le dispositif d'imagerie intravasculaire est un dispositif d'imagerie par tomographie de cohérence optique (OCT).

13. L'appareil selon l'une quelconque des revendications 1 à 12, dans lequel :
la fibre optique (120) comprend une extrémité proximale et une extrémité distale (129) ; et
la fibre optique est configurée pour émettre l'énergie électromagnétique (150) provenant de la source de lumière laser (130) depuis l'extrémité distale de la fibre optique.

14. L'appareil selon l'une quelconque des revendications 2 à 13, dans lequel le liquide (115) comprend de l'indocyanine verte (ICG) et/ou le liquide absorbe le rayonnement émis par la source de lumière laser (130).

15. L'appareil selon la revendication 13 ou 14, dans lequel la source de lumière laser (130) est configurée pour émettre de l'énergie électromagnétique (150) à une longueur d'onde d'émission ; et
le liquide (115) présente un coefficient d'absorption à la longueur d'onde d'émission ;
et/ou dans lequel l'émission d'énergie électromagnétique (150) est configurée pour permettre un contrôle temporel et spatial flexible de la génération d'ondes de choc.
